Europäisches Patentamt

European Patent Office

Office européen des brevets

(19)

(11) Numéro de publication: **0 206 890**
**B1**

(12) **FASCICULE DE BREVET EUROPEEN**

(45) Date de publication du fascicule du brevet: **29.08.90**

(21) Numéro de dépôt: **86401213.3**

(22) Date de dépôt: **05.06.86**

(51) Int. Cl.⁵: **A 23 K 1/00,** A 23 K 1/18,
A 61 K 9/52, A 61 K 9/50

(54) **Produit pour l'alimentation des ruminants et sa préparation.**

(30) Priorité: **07.06.85 FR 8508626**

(43) Date de publication de la demande:
**30.12.86 Bulletin 86/52**

(45) Mention de la délivrance du brevet:
**29.08.90 Bulletin 90/35**

(84) Etats contractants désignés:
**AT BE CH DE FR GB IT LI LU NL SE**

(56) Documents cités:
**BE-A- 838 505**
**FR-A-1 289 423**
**FR-A-1 568 930**
**FR-A-2 401 619**
**FR-A-2 514 261**
**GB-A-1 098 006**
**US-A-4 256 785**
**US-H- 100 404**

Le dossier contient des informations techniques
présentées postérieurement au dépôt de la
demande et ne figurant pas dans le présent
fascicule.

(73) Titulaire: **RHONE-POULENC NUTRITION
ANIMALE
Rue Marcel Lingot
F-03600 Commentry (FR)**

(72) Inventeur: **Sparks, Robert E.
1318 West Adams Ave
Kirkwood Missouri 63122 (US)**
Inventeur: **Mason, Norbert S.
Langton Drive
St. Louis Missouri 63105 (US)**
Inventeur: **Autant, Pierre
14 rue de Pourcheroux
F-03600 Commentry (FR)**
Inventeur: **Cartillier, André
Champfromenteau
F-03600 Commentry (FR)**
Inventeur: **Pigeon, Raymont
52 chemin Moulin du Got
F-69340 Francheville (FR)**

(74) Mandataire: **Le Pennec, Magali et al
RHONE-POULENC SANTE, Service Brevets, 20
Avenue Raymond Aron
F-92165 Antony Cédex (FR)**

# EP 0 206 890 B1

**Description**

La présente invention concerne un nouveau produit pour l'alimentation des ruminants qui est stable dans un milieu dont le pH est égal ou supérieur à 5,5 et qui permet la libération d'un principe actif dans un milieu dont le pH est inférieure ou égal à 3,5.

En particulier, lorsqu'on administre à des ruminants certaines substances biologiquement actives (médicaments, aliments enrichis), il se produit lors du passage dans le rumen une destruction enzymatique de ces substances favorisées par la temps de séjour (quelques heures à plusieurs jours) et par le pH (compris entre 5 et 6).

Il importe donc de protéger ces substances biologiquement actives par des enrobages qui soient stables à un pH supérieur ou égal à 5, c'est-à-dire qui soient stables dans la panse des ruminants, qui résistent à la dégradation par les microorganismes et qui permettent la libération des substances bioligiquement actives dans une partie de l'appareil digestif, plus particulièrement dans la caillette, dont le pH est inférieure ou égal à 3,5. Alors que la durée de protection dans la panse doit être relativement longue (plusieurs heures à quelques jours), la libération de la substance active dans la caillette doit s'effectuer en un temps relativement cort (de quelques minutes à 1 ou 2 heures).

Pour obtenir de tels résultats, il est avantageux de pouvoir disposer d'enrobages pour les substances actives dont la structure et la composition sont telles qu'ils sont insolubles dans le rumen à un pH compris entre 5 et 6, mais solubles, dispersés ou fortement gonflés dans la caillette à un pH inférieur à 3,5 pour libérer la substance active.

Il a été proposé, pour la réalisation de tels enrobages, d'utiliser, entre autres, des copolymères de l'anhydride maléique avec un autre monomère, modifiés par action d'une diamine primairetertiaire sur les groupements anhydrides, formant ainsi des groupements imides aminés qui apportent la solubilité souhaitée (brevet français FR 1 536 774). Des dérivés cellulosiques aminés sont également connus; ils sont obtenus à partir d'un dérivé non saturé de la cellulose (éther, ester) sur lequel on fait réagir un composé azoté contenant un atome d'hydrogène mobile, tel que la pipéridine, la morpholine ou une amine secondaire (brevet français FR 2081320).

Par ailleurs, dans les brevets anglais GB 1137214, australien AU 45 117 et belge BE 885 654 et le demande sud-africaine SA 70 04813 ainsi que dans les brevets français FR 2 246 572 et américain US 3 341 505 sont décrits des copolymères de:

a) un monomère éthylénique neutre comme l'acrylate ou le méthacrylate de méthyle, le styrène, l'acrylonitrile, l'acétate de vinyle, et

b) un monomère diéthylénique portant un groupement azoté basique comme l'acrylate ou le méthacrylate de diéthylaminoéthyle, l'acrylate ou le méthacrylate de morpholinoéthyle ou les vinylpyridines.

Pour enrober des aliments destinés à la nourriture des ruminants, il a été proposé d'utiliser des copolymères styrénevinylpyridine contenant des substances hydrophobes choisies parmi les acides gras contenant 10 à 32 atomes de carbone, des acides polycarboxyliques comprenant 10 à 22 atomes de carbone par groupe carboxylique qui améliorent la protection en diminuant la susceptibilité globale de la pellicule d'enrobage aux milieux aqueuse de caractère faiblement acide (brevet français FR 2401620). Dans de telles compositions d'enrobage la substance hydrophobe permet de diminuer la mouillabitité du polymère mais elle reste sans effet sur la libération du principe actif en milieu acide.

Dans le prevet français FR 2514261 a été décrit un enrobage constitué d'un copolymère sensible aux variations du pH, choisi parmi les copolymères du styrène avec les vinylpyridines, et d'un polymère non hydrosoluble insensible aux variations du pH, choisi parmi l'acétobutyrate de cellulose, l'éthylcellulose et le propionate de cellulose, ce dernier favorisant la libération de la substance active à un pH compris entre 1 et 2,5 et permettant de diminuer l'extractibilité de la substance active en milieu aqueux.

Dans la brevet français FR 2401621 a été décrite l'utilisation d'un polymère hydrophobe dans laquel est dispersée une substance soluble en milieu acide (phosphate alcalin, polymères basiques réticulés).

Dans la "Defensive Publication" publiée aux Etats-Unis sous le numéro T 100404 a été décrit un système bi-couche dans lequel la substance active est enrobée par deux couches successives constituées d'un polymère sensible aux variations du pH, tel qu'un copolymère styréne-vinylpyridine, et d'une substance hydrophobe, telle qu'un acide gras, en proportions différentes.

Le brevet FR 2401619 décrit un système dans lequel la substance active est neutralisée et enrobée, d'une seule couche constituée d'un mélange d'une substance sensible aux variations de pH et d'une substance hydrophobe.

Il a maintenant été trouves et c'est ce qui fait l'object de la présente invention, qu'un produit, constitué d'une substance active ou d'une composition contenant la substance active qui contient des inclusions ou qui est recouverte totalement ou partiellement d'une composition contenant une substance sensible aux variations du pH et dont le taux et la vitesse de gonflement sont plus importants en milieu acide qu'en milieu neutre, enrobée par une couche externe d'une composition hydrophobe, est stable dans un milieu dont le pH est égal ou supérieur à 5,5 et permet la libération du principe actif dans un milieu dont le pH est inférieur ou égal à 3,5.

Les substances sensibles aux variations du pH qui conviennent particulièrement bien pour la réalisation de l'invention sont généralement choisies parmi les polymères basiques, les sels des acides ou

2

EP 0 206 890 B1

des polyacides naturels ou synthétiques, les protéines, les polysaccharides tels que les polyglucosamines ou les alginates, ou leurs mélanges. Parmi les polymères basiques peuvent être cités les polymères contenant au moins un groupement amino basique et dont la teneur en azote basique est comprise entre 2 et 14% tels que les dérivés aminés de la cellulose, les polymères et copolymères des dérivés aminés de acides acrylique, méthacrylique et crotonique et les polymères ou copolymères du styrène ou de l'acrylonitrile avec les isomères ou les dérivés de la vinylpyridine tels que la vinyl-2 pyridine, la vinyl-4 pyridine ou la méthyl-2 vinyl-5 pyridine. Parmi les sels des acides ou des polyacides naturels ou synthétiques peuvent être cités la carbonate de calcium, le polyméthacrylate de zinc ou les polyphosphates complexes de calcium, sodium, aluminium ou magnésium. Parmi les protéines peut être citée la zéine qui est isolée du gluten de maïs. Parmi les polyglucosamines peut être cité tout particulièrement le chitosan obtenu par désacétylation de la chitine que l'on trouve en abondance dans la carapace des crustacés, ainsi que les dérivés du chitosan.

D'un intérêt tout particulier sont les polymères basiques contenant au moins un groupement amino basique et dont la teneur en azote basique est comprise entre 2 et 14%.

Ces substances sensibles aux variations du pH peuvent être utilisées seules ou en mélange ou en association avec des adjuvants tels que défini ci-après.

Les substances hydrophobes qu conviennnent particulièrement bien pour la réalisation de l'invention sont généralement choisies de telle manière que la couche externe d'enrobage présente une texture qui permet la diffusion ou la pénétration du milieu liquide extérieur. En plus d'une faible perméabilité à l'eau, les substances hydrophobes doivent avoir des propriétés mécaniques convenables telle que résistance élevée à la traction et à l'allongement et caractère filmogène.

Parmi les substances hydrophobes peuvent être citées les matières grasses, les parafinnes, les cires naturelles (cire de Carnauba, cire d'abeilles), les cires synthétiques (cire de polyéthylène), les polymères tels que le polyéthylène, le polypropylène, les polybutènes, les polyisobutènes, les polypentènes, le polystyrène, le chlorure ou le fluorure de polyvinyle, le chlorure ou le fluorure de polyvinylidène, les polyphénylènes, les oxydes de polyphénylènes, le polybutadiène, le polyisoprène, ou le polychloroprène, le polyacétate de vinyle, les dérivés cellulosiques non hydrosolubles et les latex; les substances hydrophobes peuvent être utilisées seules ou en mélange de façon à obtenir une couche externe ayant les propriétés mécaniques désirées.

Pour obtenir un enrobaged mince, il est nécessaire que la substance hydrophobe possède une viscosité convenable à l'état fondu ou en solution. Il est particulièrement intéressant que la viscosité à l'état fondu soit comprise entre 20 et 100 poises (2—10 Pa·s).

Lorsque la viscosité est trop faible, l'enrobage n'est pas satisfaisant. Lorsque la viscosité est trop élévée, la couche d'enrobage est trop épaisse et il se produit des phénomènes d'agglomération.

Dans le but d'abaisser la viscosité, il est possible de mélanger des substances liquides à température ambiante avec des substances solides: les substances liquides devant être compatibles avec le polymère.

D'un intérêt tout particulier sont les mélanges fusibles de cire de polyéthylène, de paraffine et de résines d'hydrocarbures.

Dans les compositions selon l'invention, la substance sensible aux variations du pH représente entre 0,5 et 30% du poids de la substance active ou de la composition contenant la substance active.

Dans les compositions selon l'invention, la couche externe d'enrobage, dont l'épaisseur moyenne peut varier de 5 à 200 µm selon la taille du granulé à enrober, représente 1 à 50% du poids total de la composition.

Selon la présente invention, les compositions peuvent contenir des adjuvants dont la rôle est de faciliter la mise en oeuvre des techniques de préparation de ces compositions ou d'améliorer les caractéristiques physico-chimiques. Il peut être avantageux d'ajouter des agents plastifiants (triacétine, propylèneglycol), des agents lubrifiants (stéarate de magnésium), des agents liants (polyvinylpyrrolidone, alcool polyvinylique, gélatine; des agents antistatiques (triglycérides à chaînes polyoxyéthylénées), des agents anti-mortants (silice, carbonate de calcium), des agents fongicides, des agents émulsifiants (esters de sorbitan oxyéthylénés, sucroglycérides), des agents de compatibilisation (gommes naturelles ou seminaturelles telles que les alginates, la gomme adragante, les pectines, les carraghénates, la gomme xanthane), des éthers cellulosiques (carboxyméthyl-, méthyl- ou hydroxypropylcellulose) de charges minérales (sels minéraux), des sucres, des amidons ou des protéines. Ces dérivés adjuvants ne représentent généralement que quelques pourcents en poids de l'enrobage.

Les substances actives entrant dans les produits selon l'invention sont des substances thérapeutiques ou nutritives diverses telles que des médicaments, des vitamines (vitamine A, vitamine E) ou des aminoacides (lysine, méthionine) destinées à être administrées par voie orale à des ruminants. Les substances thérapeutiques ou nutritives se présentent généralement sous forme solide; lorsque les substances thérapeutiques ou nutritives se présentent sous forme liquide, elles peuvent être éventuellement adsorbées sur un support inerte tel que la silice, les silicates les alumines, les aluminates, les silicoaluminates et les amidons.

Les nouveaux produits selon la présente invention sont, de préférence, des granulés, généralement sphériques ou cyclindriques, dont le diamètre moyen est compris entre 0,05 et 5 mm.

Les nouveaux produits selon l'invention peuvent être préparés par application des techniques connues de granulation et d'enrobage.

3

EP 0 206 890 B1

d'une manière générale, il est nécessaire d'effectuer un mélange ou un pré-enrobage total ou partiel de la substance active ou de la composition contenant la substance active avec la substance sensible aux variations du pH puis d'enrober le produit ainsi obtenu, généralement sous forme de granulés, avec la substance hydrophobe.

Le mélange de la substance active avec la substance sensible aux variations du pH peut être effectué en mélangeant la substance active finement divisée avec la substanace sensible aux variations du pH finement divisée puis à granuler le produit obtenu.

L'enrobage partiel ou total de la substance active avec la substance sensible aux variations du pH peut être effectué selon les techniques habituelles d'enrobage telle que l'encapsulation en lit fluidisé, le trempage ou la coacervation.

Pour effectuer l'enrobage de la substance active, mélangée avec la substance sensible aux variations du pH ou pré-enrobée totalement ou partiellement avec la substance sensible aux variations du pH, avec la substance hydrophobe différentes techniques peuvent être utilisées.

Il est possible d'effectuer l'enrobage en lit fluidisé, par trempage, par adsorption en milieu liquide ou par coacervation.

Il est également possible d'effectuer l'enrobage par la substance hydrophobe fondue ou en solution en projetant une suspension de la substance active pré-traitée dans la substance fondue ou en solution dans un solvant organique convenable dans lequel la substance active pré-traitée n'est pas soluble sur un disque plat ou concave comportant éventuellement des rainures tournant à une vitesse déterminée et chauffée par de l'air chaud généralement à une température supérieure à 20°C à la température de solidification de la composition hydrophobe. Généralement la substance active prétraitée est dispersé dans 2 fois son poids de composition hydrophobe.

L'excès de composition hydrophobe forme des petites particules qui restent à proximité du disque alors que les particules de substance active enrobée sont éjectées plus loin. Il en résulte que la séparation des particules de substance active enrobée et des particules de composition hydrophobe se faite systématiquement au cours de la mise en oeuvre du procédé. Par ailleurs, l'excès de composition hydrophobe peut être recyclé.

Les exemples suivants montrent comment l'invention peut être mise en pratique.

Exemple 1

On pré-enrobe de la méthionine, sous forme de noyaux sphériques contenant 98% de méthionine et avant un diamètre compris entre 0,5 et 0,63 mm par un copolymère vinyl-2 pyridine-styrène (70—30) en utilisant la technique du lit fluidisé pour obtenir des granulés pré-enrobés pour lesquels le taux de pré-enrobage (poids d'enrobage/poids de moyaux × 100) est de 4.25% et l'épaisseur de la couche pré-enrobante est voisine de 5 microns.

Exemple 2

On opère comme dans l'exemple 1 mais de façon à obtenir une méthionine pré-enrobée pour laquelle le taux de pré-enrobage est de 7% et l'épaisseur de la couche de pré-enrobage est voisine de 8 microns.

Exemple 3

On opère comme dans l'exemple 1 mais de façon à obtenir une méthionine pré-enrobée pour laquelle le taux de pré-enrobage est de 13,5% et l'épaisseur de la couche de pré-enrobage est voisine de 15 microns.

Exemple 4

On enrobe 52 g de granulés pré-enrobés obtenus à l'exemple 1 en projetant sur un disque tournant à 21150 tours/minute une suspension de ces granulés dans 60 g d'une composition hydrophobe constituée de cire Bareco C 1035 (30 g), paraffine dont le point de fusion est compris entre 58 et 63°C (10 g), polyéthylène USI NA 601 (20 g) et de 25 cm3 d'un mélange octane-heptane (1-1 en volumes) à une température de 90°C.

On obtient ainsi 12,6 g de granulés de méthionine enrobée contenant 64,7% de méthionine et dont le diamètre est compris entre 0,5 et 1 mm. Pour 83% des granulés obtenus, le diamètre est compris entre 0,59 et 0,86 mm.

Exemple 5

On opère comme dans l'exemple 4 mais à partir de 52 g de méthionine pré-enrobée obtenue à l'exemple 2 en opérant à une température de 96°C, la vitesse de rotation du disque étant de 2160 tous/minutes.

On obtient 15,9 g de granulés de méthionine enrobée contenant 61,6% de méthionine dont le diamètre est compris entre 0,5 et 1 mm.

Exemple 6

On opère comme dans l'exemple 4 mais à partir de 52 g de méthionine pré-enrobée obtenue à l'exemple 3 en opérant à une température de 98°C, la vitesse de rotation du disque étant de 2160 tous/minute.

On obtient 12,1 g de granulés de méthionine enrobée contenant 60,1% de méthionine dont le diamètre est compris entre 0,5 et 1 mm.

4

### Exemple 7

On enrobe 52 g de méthionine pré-enrobée obtenue à l'exemple 1 par 100 g d'une composition hydrophobe constituée de polywax 500 (50 g), paraffine (30 g), polyéthylène USI NA 597 (20 g) en opérant à une température de 124°C, la vitesse de rotation du disque étant de 2160 tours/minute.

On obtient ainsi 17,6 g de granulés de méthionine enrobée contenant 65,5% de méthionine et dont le diamètre est compris entre 0,5 et 1 mm.

### Exemple 8

On opère comme dans l'exemple 7 mais à partir de 52 g de méthionine pré-enrobée obtenue à l'exemple 2 et de 100 g de composition hydrophobe en opérant à 132°C la vitesse de rotation du disque étant de 2160 tours/minute.

On obtient 17,7 g de granulés de méthionine enrobée contenant 61,6% de méthionine dont le diamètre est compris entre 0,5 et 1 mm.

### Exemple 9

On opère comme dans l'exemple 7 mais à partir de 52 g de méthionine pré-enrobée obtenue à l'exemple 3, et de 100 g de composition hydrophobe en opérant à une température de 121°C, la vitesse de rotation du disque étant de 2160 tours/minute.

On obtient 17 g de granulés de méthionine enrobée contenant 59,3% de méthionine dont le diamètre est compris entre 0,5 et 1 mm.

### Exemple 10

On enrobé 150 g de méthionine pré-enrobée obtenue à l'exemple 1 par 300 g d'une composition constituée de polywax 500 (150 g), paraffine (90 g), polyéthylène USI NA 597 (60 g) en opérant à une température de 124°C, la vitesse de rotation du disque étant de 2160 tours/minute.

On obtient ainsi 82,6 g de granulés de méthionine enrobée contenant 63% de méthionine dont le diamètre est compris entre 0,5 et 1 mm.

### Exemple 11

On opère comme dans l'exemple 10 mais à partir de 155 g de méthionine pré-enrobée obtenue à l'exemple 2 et de 300 g de composition hydrophobe en opérant à une température de 118°C, la vitesse de rotation du disque étant de 2160 tours/minute.

On obtient ainsi 85 g de granulés de méthionine enrobée contenant 64,8% de méthionine dont le diamètre est compris entre 0,5 et 1 mm.

### Exemple 12

On opère comme dans l'exemple 10 mais à partir de 165 g de méthionine pré-enrobée obtenue à l'exemple 3 et de 300 g de composition hydrophobe en opérant à une température de 102°C, la vitesse de rotation du disque étant de 2160 tours/minute.

On obtient ainsi 51,2 g de granulés de méthionine enrobée contenant 55,3% de méthionine dont le diamètre est compris entre 0,5 et 1 mm.

### Exemple 13

Des granulés sphériques de méthionine d'un diamètre compris entre 0,5 et 0,63 mm sont pré-enrobés, en utilisant la technique sur lit fluidisé par 7% de copolymère de méthacrylate de diméthylaminoacétate (Eudragit E, N. D. ROHM PHARMA) de telle manière que la couche de pré-enrobage ait une épaisseur voisien de 8 microns.

Les granulés ainsi obtenus sont enrobés par un mélange fondu de polywax 500 (50), paraffine (30) et polyéthylène USI NA 597 (20). On obtient ainsi des granulés enrobés contenant 56% de méthionine et dont le diamètre est compris entre 0,5 et 1 mm.

Pour mettre en évidence la sensibilité des produits selon l'invention aux variations du pH, des tests sont utilisés qui permettent de mesurer le relargage de la matière active en fonction du temps à différentes valeurs du pH.

Plus particulièrement le relargage de la substance active sous forme de granulés est examiné en agitant, dans des conditions déterminées, une quantité connue de granulés dans le milieu tamponné maintenu à pH constant à une température de 40°C. On compare les vitesses de relargage d'un échantillon soumis à différentes valeurs du pH, notamment à pH = 6 et à pH = 2.

Pour les produits qui font l'objet des exemple 1 à 9, les résultats obtenus sont rassemblés dans le tableau 1.

TABLEAU 1

| EXEMPLES | RELARGAGE APRES 1/2 H. à | | RELARGAGE APRES 2 H. à | | RELARGAGE APRES 3 H. à | | RELARGAGE APRES 24 H. à | |
|---|---|---|---|---|---|---|---|---|
| | pH 6 | pH 2 | pH 6 | pH 2 | pH 6 | pH 2 | pH 6 | pH 2 |
| 1 | 92,7 | 100 | 100 | 100 | 100 | 100 | 100 | 100 |
| 2 | 48,9 | 100 | 98,8 | 100 | 100 | 100 | 100 | 100 |
| 3 | 11,3 | 98,1 | 55,4 | 100 | 68,8 | 100 | 96,5 | 100 |
| 4 | 1,3 | 42,5 | 8,4 | 91,5 | 15,2 | 94,9 | | |
| 5 | 1,3 | 95,0 | 4,6 | 100 | 5,6 | 100 | | |
| 6 | 1,3 | 89,9 | 2,5 | 100 | 3,6 | 100 | 18,1 | 100 |
| 7 | 1,3 | 48,8 | 2,5 | 91,6 | 3,6 | 93,9 | 61,0 | 99,0 |
| 8 | 0,6 | 41,2 | 2,4 | 87,8 | 2,5 | 91,3 | 40,4 | 98,4 |
| 9 | 1,3 | 47,8 | 1,9 | 77,2 | 2,5 | 79,5 | 20,1 | 93,3 |
| 10 | 1,9 | 4,5 | 1,3 | 18,4 | 2,4 | 23,0 | 49,3 | 74,0 |
| 11 | 2,5 | 15,3 | 2,5 | 55,3 | 2,5 | 62,2 | 40,7 | 83,7 |
| 12 | 1,2 | 51,9 | 1,9 | 91,8 | 2,9 | 96,3 | 21,9 | 100 |
| 13 | 2 | 30 | 5 | 55 | 10 | 62 | | |

L'efficacité in vivo des nouveaux produits selon la présente invention peut être mise en évidence dans les tests suivants:

1) Test de la méthioninémie
Un dose régulière de méthionine rapportée au poids métabolique de l'animal est donnée à ingérer quotidiennement pendant 7 jours à une brebis. Des prises de sang sont effectuées les 6ème et 7ème jours et la méthionine sanguine est déterminée par la méthode de Stein et Moore J. Biol. Chem. *192*, 663 (1951). On compare, dans des conditions de supplémentation indentiques, le méthioninémies provoquées par la méthionine protégée ou non protégée.
Les résultats sont rassemblés dans le tableau II.

TABLEAU II

| PRODUITS | SUPPLEMENTATION (EQUIVALENT METHIONINE en g/kg de POIDS META-BOLIQUE par JOUR) | METHIONINEMIE (mg de METHIONINE par g de SANG) |
|---|---|---|
| Méthionine non protégée | 0,38 | 0,36 |
| exemple 7 | 0,38 | 2,0 |
| exemple 8 | 0,38 | 1,50 |
| exemple 9 | 0,30 | 1,50 |

2) Test de la concentration en méthionine libre dans le jus duodénal.

De la méthionine est donnée à ingérer en doses régulières, dans des conditions standards, à des brebis fistulées du duodénum. Les 4ème et 5ème de la supplémentation on effectue des prélèvements du jus duodénal dans lequel la méthionine libre est dosée.

Les résultats sont rassemblés dans le tableau III.

TABLEAU III

| PRODUIT | SUPPLEMENTATION g de METHIONINE/JOUR | METHIONINE LIBRE (mg par kg de JUS DUODENAL) |
|---|---|---|
| Méthionine non protégée | 10 | 51 |
| exemple 7 | 10 | 228 |
| exemple 8 | 10 | 362 |
| exemple 9 | 10 | 241 |

**Revendications**

1. Un nouveau produit pour l'alimentation des ruminants qui est stable dans un milieu dont le pH est égal ou supérieur à 5,5 et qui permet la libération d'un principe actif dans un milieu dont le pH est inférieur ou égal à 3,5 caractérisé en ce qu'il est constitué d'une substance active ou d'une composition contenant la substance active qui contient des inclusions ou qui est recouverte totalement ou partiellement d'une composition contenant une substance sensible aux variations du pH dont le taux et la vitesse de gonflement sont plus importants en milieu acide qu'en milieu neutre, enrobée par une couche d'une composition hydrophobe.

2. Un nouveau produit selon la revendication 1 caractérisé en ce que la substance sensible aux variations du pH est choisie parmi les polymères basiques, les sels des acides ou polyacides naturels ou synthétiques, les protéines, les polysaccharides ou leurs mélanges.

3. Un nouveau produit selon la revendication 1 caractérisé en ce que la substance sensible aux variations du pH est choisie parmi les polymères ou copolymères contenant au moins un groupement amino basique et dont la teneur en azote est comprise entre 2 et 14%.

4. Un nouveau produit selon la revendication 1 caractérisé en ce que la substance sensible aux variations du pH est choisie parmi les copolymères du styrène avec les isomères ou les dérivés de la vinylpyridine.

5. Un nouveau produit selon la revendication 1 caractérisé en ce que la couche enrobante hydrophobe

7

# EP 0 206 890 B1

est constituée de substances hydrophobes choisies parmi les matières grasses, les paraffines, les cires naturelles, les cires synthétiques et les polymères dérivés de l'éthylène, de l'isobutylène ou de l'acétate de vinyle et les dérivés cellulosiques non hydrosolubles, seules ou en mélange.

6. Un nouveau produit selon la revendication 1 caractérisé en ce que la substance active est choisie parmi les médicaments, les vitamines et les aminoacides essentiels.

7. Un nouveau produit selon la revendication 1 caractérisé en ce que la substance active est la lysine ou la méthionine.

8. Un procédé de préparation d'un produit selon la revendication 1 caractérisé en ce que l'on effectue un mélange ou un pré-enrobage total ou partiel de la substance active avec la substance sensible aux variations du pH puis enrobe le produit ainsi obtenu avec la substance hydrophobe.

## Patentansprüche

1. Neues Futtermittel für Wiederkäuer, das in einem Milieu stabil ist, dessen pH gleich oder größer als 5,5 ist, und das die Freisetzung eines aktiven Bestandteils in einem Milieu zuläßt, dessen pH-Wert kleiner oder gleich 3,5 ist, dadurch gekennzeichnet, daß es aus einer aktiven Substanz oder einem die aktive Substanz enthaltenden Präparat, die Einschlüsse aus einem Material enthalten oder vollständig oder teilweise mit einem Material überzogen sind, das eine gegenüber pH-Wert-Variationen empfindliche Substanz enthält, die in einem Ausmaß und mit einer Geschwindigkeit quillt, die in saurem Milieu größer als in neutralem Milieu ist, umhüllt von einer Schicht aus einem hydrophoben Material, besteht.

2. Neues Produkt nach Anspruch 1, dadurch gekennzeichnet, daß die gegenüber pH-Wert-Variationen empfindliche Substanz ausgewählt ist aus den basischen Polymeren, den Salzen von natürlichen oder synthetischen Säuren oder Polysäuren, den Proteinen, den Polysacchariden oder ihren Mischungen.

3. Neues Produkt nach Anspruch 1, dadurch gekennzeichnet, daß die gegenüber pH-Wert-Variationen empfindliche Substanz ausgewählt ist aus den Polymeren oder Copolymeren, die zumindest eine basische Aminogruppe enthalten und deren Stickstoffgehalt zwischen 2 und 14% liegt.

4. Neues Produkt nach Anspruch 1, dadurch gekennzeichnet, daß die gegenüber Variationen des pH-Werts empfindliche Substanz ausgewählt ist aus den Copolymeren von Styrol mit den Vinylpyridinisomeren oder -derivaten.

5. Neues Produkt nach Anspruch 1, dadurch gekennzeichnet, daß die hydrophobe Umhüllungsschicht aus hydrophoben Substanzen besteht, ausgewählt aus den Fetten, den Paraffinen, den natürlichen Wachsen, den synthetischen Wachsen, den polymeren Äthylen-, Isobutylen- oder Vinylacetatderivaten und den nicht wasserlöslichen Cellulosederivaten, allein oder in Mischung.

6. Neues Produkt nach Anspruch 1, dadurch gekennzeichnet, daß die aktive Substanz ausgewählt ist aus den Medikamenten, den Vitaminen und den essentiellen Aminosäuren.

7. Neues Produkt nach Anspruch 1, dadurch gekennzeichnet, daß die aktive Substanz Lysin oder Methionin ist.

8. Verfahren zur Herstellung eines Produkts nach Anspruch 1, dadurch gekennzeichnet, daß man eine Mischung oder eine vollständige oder teilweise Vorumhüllung der aktiven Substanz mit der gegenüber pH-Wert-Variationen empfindlichen Substanz ausführt und dann das so erhaltene Produkt mit der hydrophoben Substanz umhüllt.

## Claims

1. A new product for feeding ruminants, which is stable in a medium in which the pH is equal to or above 5.5 and which permits the release of an active principle in a medium in which the pH is below or equal to 3.5, characterized in that it consists of an active substance or of a composition containing the active substance which contains inclusions of, or which is completely or partially covered by, a composition containing a substance which is sensitive to pH variations whose extent and rate of swelling are greater in an acidic medium than in a neutral medium, said active substance or composition containing active substance being coated with a layer of a hydrophobic composition.

2. A new product according to Claim 1, characterized in that the substance which is sensitive to pH variations is chosen from basic polymers, salts of natural or synthetic acids or polyacids, proteins, polysaccharides or mixtures of these.

3. A new product according to Claim 1, characterized in that the substance which is sensitive to pH variations is chosen from polymers or copolymers containing at least one basic amino group and having a nitrogen content of between 2 and 14%.

4. A new product according to Claim 1, characterized in that the substance which is sensitive to pH variations is chosen from copolymers of styrene with isomers or derivatives of vinylpyridine.

5. A new product according to Claim 1, characterized in that the hydrophobic coating layer consists of hydrophobic substances chosen from fats, paraffin waxes, natural waxes, synthetic waxes and polymers derived from ethylene, isobutylene or vinyl acetate, and non-water-soluble cellulose derivatives, alone or mixed.

6. A new product according to Claim 1, characterized in that the active substance is chosen from drugs, vitamins and essential amino acids.

8

7. A new product according to Claim 1, characterized in that the active substance is lysine or methionine.

8. A process for preparing a product according to Claim 1, characterized in that a mixture is made, or a complete or partial pre-coating is performed, of the active substance with the substance which is sensitive to pH variations, and the product thereby obtained is then coated with the hydrophobic substance.